(19) Europäisches Patentamt
European Patent Office
Office européen des brevets

(11) Publication number: 0 168 881 B1

# (12) EUROPEAN PATENT SPECIFICATION

(45) Date of publication of patent specification: 04.01.89

(51) Int. Cl.$^4$: C 07 K 5/06, C 07 D 493/08, A 23 L 1/236

(21) Application number: 85201092.5

(22) Date of filing: 05.07.85

## (54) Oxa-fenchyl esters and amides of alpha-l-aspartyl-d-phenylglycine.

(30) Priority: 13.07.84 US 630457

(43) Date of publication of application: 22.01.86 Bulletin 86/04

(45) Publication of the grant of the patent: 04.01.89 Bulletin 89/01

(84) Designated Contracting States: AT BE CH DE FR GB IT LI NL

(56) References cited:
EP-A-0 034 876
EP-A-0 069 811
EP-A-0 074 249
US-A-3 907 766
US-A-3 972 860

(73) Proprietor: THE PROCTER & GAMBLE COMPANY
One Procter & Gamble Plaza
Cincinnati Ohio 45202 (US)

(72) Inventor: Janusz, John Michael
2068 John Gray Road
Fairfield Ohio 45015 (US)
Inventor: Gardlik, John Michael
6936 Parkview Drive
Cincinnati Ohio 45224 (US)

(74) Representative: Brooks, Maxim Courtney et al
Procter & Gamble (NTC) Limited Whitley Road
Longbenton
Newcastle-upon-Tyne NE12 9TS (GB)

EP 0 168 881 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present application relates to oxa-fenchyl and like esters and amides of *alpha*-L-aspartyl-D-phenylglycine useful as high intensity sweeteners.

Sweeteners are used in a variety of orally ingested products. For example, sweeteners are an important component of cakes, cookies, chewing gum, dentifrices and the like. Sweeteners are a particularly important ingredient in beverages. In terms of volume, carbonated beverages use more sweeteners than any other sweetened product category.

The most widely used sweetener for food, and especially beverage products, is sucrose. Sucrose is safe, naturally occurring, and has a high sweetness quality in terms of a pure, quick onset of sweetness with no aftertaste or undertaste. However, the normal usage of sucrose provides significant caloric load which is undesirable for those persons on weight control or reduction programs. Also, those persons who have diabetes must carefully control their intake of sucrose to avoid problems associated with the disease. Sucrose is also cariogenic so that it cannot be used in dentifrices and is indesirable in chewing gums. Additionally, and perhaps little realized, for the amount of sweetness delivered, sucrose can be expensive relative to other sweeteners such as saccharin, especially when used in carbonated beverages.

The drawbacks of sucrose, including its expensive, have led those in the beverage industry to seek substitute sweeteners. One particularly important quality sought in such sweeteners is high sweetness intensity. Sweetness intensity can affect not only the safety profile and caloric value of the sweetener, but also its cost in terms of sucrose equivalent sweetness. However, the inability to predict that a given compound is sweet, and particularly that it has high sweetness intensity, makes the search for suitable substitute sweeteners a "hit-or-miss" proposition.

Such unpredictability is especially true for the currently popular L-aspartic acid derived sweeteners represented by the following formula:

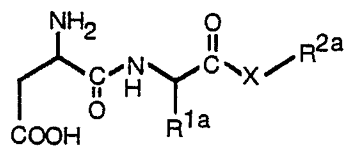

where X is O (ester) or NH (amide). Various theories have been proposed for what imparts sweetness to these particular molecules. However, the current belief is that groups $R^{1a}$ and $R^{2a}$ need to be dissimilar in size for greatest sweetness intensity, i.e. one group large or bulky, the other group small. See Goodman et al., "Peptide Sweeteners: A Model for Peptide and Taste Receptor Interactions," *Proc. 15th Eur. Pep. Symp.,* (1974), pp. 271—78; Sukehiro et al., "Studies on Structure-Taste Relationships of Aspartyl Peptide Sweeteners: Syntheses and Properties of L-Aspartyl-D-Alanine Amides," *Science of Human Life,* Vol. 11, (1977), pp. 9—16. It also appears that when $R^{1a}$ is the large or bulky group, the stereochemical configuration generally needs to be L, L for sweetness. See US—A—3,972,860 to Moriarty et al., issued August 3, 1976 (L-aspartyl-L-phenylglycine lower alkyl esters are sweet); US—A—3,492,131 to Schlatter, issued January 27, 1970 (L-aspartyl-L-phenylalanine lower alkyl esters are sweet). Conversely, when $R^{1a}$ is the small group, the stereochemical configuration generally needs to be L, D for sweetness. See US—A—4,411,925 to Brennan et al., issued October 25, 1983 (L-aspartyl-D-alanine amides are sweet); Ariyoshi et al., "The Structure-Taste Relationships of the Dipeptide Esters Composed of L-Aspartic Acid and Beta-Hydroxyamino Acids," *Bull. Chem. Soc. Jap.,* Vol. 47, (1974), pp. 326—30 (L-aspartyl-D-serine esters are sweet). Even with these guidelines, the sweetness intensity of these L-aspartic acid derived sweeteners can vary greatly depending upon which combination of $R^{1a}$ and $R^{2a}$ groups are selected. Compare US—A—4,411,925, *supra* (X is NH, $R^{1a}$ is methyl group, $R^{2a}$ is 2,6-dimethylcyclohexyl group, sweetness intensity is 600 times that of sucrose), with US—A—3,907,766 to Fujino et al., issued September 23, 1975 (X is 0, $R^{1a}$ is methyl ester group, $R^{2a}$ is fenchyl group, sweetness intensity is 22,200—33,200 times that of sucrose).

For beverage use, the substitute sweetener must be sufficiently soluble and hydrolytically stable. Most carbonated beverages have a pH of from about 2.5 to about 4.8. Useful sweeteners in such beverages must therefore be relatively resistant to acid catalyzed breakdown. Otherwise, the beverage can quickly lose its sweetness or possibly have undesirable off-flavors imparted to it. As in the case of sweetness intensity, it can be difficult to predict whether a given sweetener will be hydrolytically stable, especially in an acidic environment.

Other factors are also important in providing a useful substitute sweetener. To obtain approval for food or beverage use, the substitute sweetener must be safe in terms of acute toxicity as well as long-term effects from continued use. The substitute sweetener should also desirably approach sucrose in terms of sweetness quality, as well as have a relatively quick onset and short duration of sweetness. Finally, to be classified as a non-caloric sweetener, the substitute sweetener (or metabolic products thereof) should provide minimal or no caloric value at normal usage levels.

The most widely used substitute sweetener at present is saccharin, in particular its sodium salt. Saccharin has a relatively high sweetness intensity (about 300 times that of sucrose) and is relatively

inexpensive in providing sucrose equivalent sweetness in carbonated beverages. However, saccharin also provides an undesirable lingering bitter aftertaste.

Besides saccharin, a number of the L-aspartic acid derived amides have been proposed as suitable substitute sweeteners. The most prominent examples are the *alpha*-L-aspartyl-L-phenylalanine lower alkyl esters, in particular the methyl ester known as aspartame. Aspartame has been approved for use in dry foods and beverages, and has recently been approved for use in aqueous beverage systems such as carbonated beverages. The sweetness intensity of aspartame is about 150—200 times that of sucrose with a sweetness quality approaching that of sucrose. The caloric value of aspartame is also relatively minimal at normal usage levels. However, aspartame is hydrolytically unstable in most carbonated beverages. Perhaps more important to the beverage industry, aspartame is extremely expensive in terms of sucrose equivalent sweetness delivered.

The search therefore continues for substitute sweeteners which are: (1) inexpensive in terms of sucrose equivalent sweetness; (2) are hydrolytically stable in carbonated beverage systems; (3) are safe; (4) have satisfactory taste quality; and (5) provide minimal caloric value.

## BACKGROUND ART

A. *L-aspartyl-L-phenylglycine esters*

*US—A—3,972,860 to Moriarty et al., issued August 3, 1976,* discloses L-aspartyl-L-phenylglycine lower alkyl ester sweeteners. The preferred methyl ester is disclosed as having a sweetness intensity of from 100—1000 times that of sucrose. See also Goodman et al., "Peptide Sweeteners: A Model for Peptide and Taste Receptor Interactions," *Proc. 15th Eut. Pep. Symp.,* (1975), pp. 271—78, which discloses that the methyl ester of L-aspartyl-L-phenylglycine is "quite sweet."

B. *Peptides Containing D-phenylglycine*

*US—A—4,183,909 to Schon et al. issued January 15, 1980,* discloses phenylglycine-containing peptides which greatly increase gastric acid secretion when administered intravenously. One of the precursors of these peptides is the beta-*tert*-butyl ester of L-aspartyl-D-phenylglycine hydrochloride (Example 1, Step 4).

C. *L-aspartyl-D-alanine amides*

*US—A—4,411,925 to Brennan et al. issued October 23, 1983,* discloses L-apartyl-D-alanine amide sweeteners. These amides have the formula:

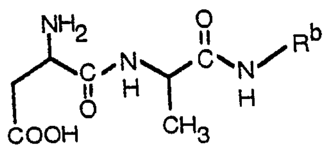

wherein $R^b$ is a branched hydrocarbyl group, including fenchyl (320 times as sweet as sucrose). The highest intensity sweeteners include those where $R^b$ is 2,5-dimethylcyclopentyl (520 times that of sucrose), 2,6-dimethylcyclohexyl (600 times that of sucrose), dicyclopropylcarbinyl (1200 times that of sucrose), 2,2,4,4-tetramethylthietan-3-yl (2000 times that of sucrose), or 2,2,4,4-tetramethyl-1,1-dioxothietan-3-yl (1000 times that of sucrose). See also Sukehiro et al., "Studies on Structure-Taste Relationships of Aspartyl Peptide Sweeteners: Syntheses and Properties of L-Aspartyl-D-Alanine Amides," *Science of Human Life,* Vol. 11, (1977), pp. 9—16, which discloses L-aspartyl-D-alanine amide sweeteners (10 to 125 times that of sucrose) wherein $R^b$ is $C_2$—$C_4$ alkyl or cyclohexyl.

D. *L-aspartyl-aminomalonic acid diesters*

*US—A—3,907,766 to Fujino et al., (assigned to Takeda Chemical Industries, Ltd.), issued September 23, 1975* discloses L-aspartyl-aminomalonic diester sweeteners. These diesters have the formula:

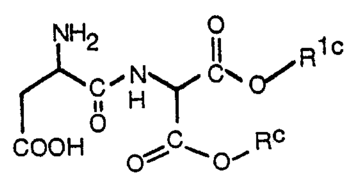

wherein $R^{1c}$ is fenchyl and $R^c$ is methyl (22, 200—33,200 times that of sucrose) or ethyl (4200—5400 times that of sucrose). Fujino et al., "Structure-Taste Relationships of L-aspartyl-aminomalonic Acid Diesters," *Chem. Pharm. Bull.,* Vol. 24 (1976), pp. 2112—17, suggests that the L-aspartyl-L-aminomalonic acid diester is the sweet one. See page 2116. See also US—A—3,801,563 to Nakajima et al. (assigned to Takeda Chemical Industries, Ltd.), issued April 2, 1974, which discloses other L-aspartyl-amino-malonic acid diesters containing branched or cyclic alkyl ester groups.

E. *L-aspartyl-D-amino acid esters*

*Mazur et al., "Synthetic Sweeteners:Aspartyl Dipeptide Esters from L- and D-alkylglycines," J. Med. Chem., Vol. 16, (1973), pp. 1284—87,* discloses sweetness intensity testing of isopropyl esters of L-aspartyl-D-amino acids. These esters have the formula:

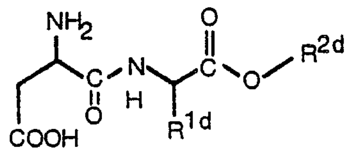

wherein $R^{2d}$ is isopropyl and $R^{1d}$ is a $C_1$—$C_4$ alkyl group. The sweetness intensity of the particular esters ranges from 0—170 times that of sucrose.

*Ariyoshi et al., "The Structure-Taste Relationships of the Dipeptide Esters Composed of L-aspartic Acid and Beta-hydroxyamino Acids," Bull. Chem. Soc. Jap., Vol. 47, (1974), pp. 326—30,* discloses sweetness intensity testing of $C_1$—$C_4$ alkyl or cyclohexyl esters of L-aspartyl-D-amino acids. These esters have the formula:

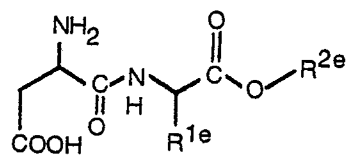

wherein $R^{2e}$ is a $C_1$—$C_4$ alkyl or cyclohexyl group, and $R^{1e}$ is a $C_1$—$C_2$ alkyl or hydroxyalkyl group. The D-amino acids used include D-serine ($R^{1e}$ = hydroxymethyl); D-threonine ($R^{1e}$ = hydroxyethyl), D-allothreonine ($R^{1e}$ = a-hydroxyethyl), and D-2-aminobutyric acid ($R^{1e}$ = ethyl). The sweetness intensity of the particular esters can range from 6—320 times that of sucrose.

*Ariyoshi "The Structure-Taste Relationships of Aspartyl Dipeptide Esters," Agr. Biol. Chem., Vol. 40, (1976), pp. 983—92,* discloses sweetness intensity testing of $C_1$—$C_3$ alkyl or cyclohexyl esters of L-aspartyl-D-amino acids. These esters have the formula:

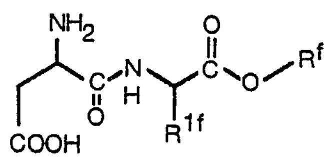

wherein $R^f$ is a $C_1$—$C_3$ alkyl or cyclohexyl group, and $R^{1f}$ is a $C_1$—$C_3$ alkyl or hydroxyalkyl, or benzyl group. The methyl ester of L-aspartyl-D-phenylalanine is disclosed to be bitter.

See also US—A—3,492,131 to Schlatter (assigned to G. D. Searle & Co.), issued January 27, 1970, which states that the L-aspartyl-D-phenylalanine esters are not sweet.

## DISCLOSURE OF THE INVENTION

The present invention relates to certain *alpha*-L-aspartyl-D-phenylglycine esters and amides useful as sweeteners. These esters and amides include the non-toxic salts and have the formula:

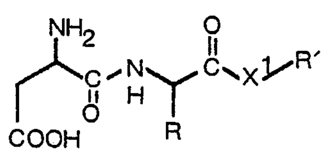

wherein the ester or amide is the L, D stereochemical isomer; wherein $X^1$ is O or NH; wherein R is a phenyl group having the formula:

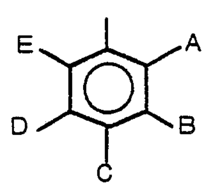

wherein A, B, C, D and E are H, OH, F, Cl, Br, or $C_1$—$C_4$ alkyl, hydroxyalkyl or alkoxy; and wherein R′ is selected from bicyclic radicals having formulas (a), (b) and (c):

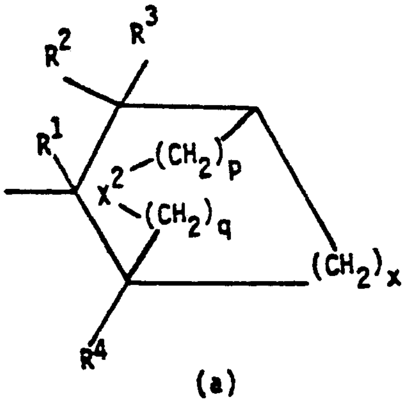

(a)

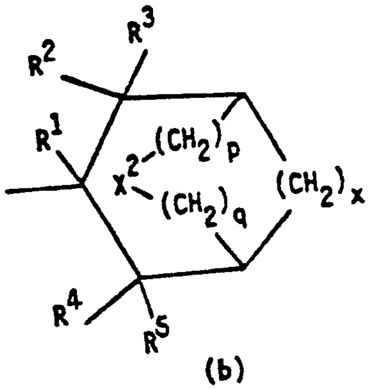

(b)

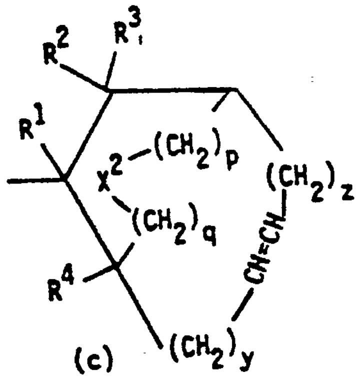

(c)

wherein $R^1$, $R^2$, $R^3$, $R^4$ and $R^5$ are H, or $C_1$—$C_4$ alkyl, hydroxyalkyl or alkoxy; provided that at least one of $R^2$, $R^3$, $R^4$ and $R^5$ are $C_1$—$C_4$ alkyl, hydroxyalkyl or alkoxy, $X^2$ is O; p and q are 0, 1, 2 or 3 and the sum of p + q is not greater than 3; x is 1, 2 or 3; y and z are 0, 1 or 2 and the sum of y + z is not greater than 2.

These *alpha*-L-aspartyl-D-phenylglycine esters and amides are more hydrolytically stable in carbonated beverages than aspartame. Also, certain of these esters and amides have sufficiently high sweetness intensity so as to be relatively inexpensive in terms of sucrose equivalent sweetness. Based on available data for the expected metabolites, it is believed that these esters and amides are safe for use in food and beverage systems, and will provide minimal caloric value at normal usage levels. The taste quality of these sweeteners is also satisfactory.

A. *Alpha-L-aspartyl-D-phenylglycine esters and amides*

The esters and amides of the present invention have the formula:

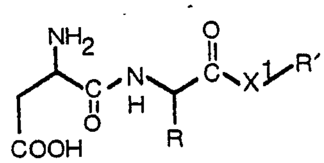

It has been determined that the L,D stereochemical isomer imparts the sweetness character to these esters and amides. However, minor amounts of the D,L, L,L and D,D stereochemical isomers can be tolerated without adversely affecting the taste quality of L,D stereochemical isomer. Such diastereomeric mixtures typically comprise at least 50% of the L,D stereochemical isomer, preferably at least 70% of the L,D isomer, and most preferably at least 95% of the L,D isomer.

The esters or amides of the present invention can be in the form of non-toxic salts. As used herein, "non-toxic salts" means salts of the present esters and amides which are physiologically acceptable for ingestion. Such salts include both cationic and acid addition salts of these esters and amides. By "cationic salts" is meant those salts formed by neutralization of the free carboxylic acid group of the instant esters and amides by bases of physiologically acceptable metals, ammonia and amines. Examples of such metals are sodium, potassium, calcium and magnesium. Examples of such amines are n-methyl-glucamine and ethanolamine. By "acid addition salts" is meant those salts formed between the free amino group of the instant esters and amides and a physiologically acceptable acid. Examples of such acids are acetic, benzoic, hydrobromic, hydrochloric, citric, fumaric, gluconic, lactic, maleic, malic, sulfuric, sulfonic, nitric, phosphoric, saccharic, succinic and tartaric acids.

The compounds of the present invention can be in the form of either esters or amides ($X^1$ is O or NH). The amides are desirable from the standpoint of having greater hydrolytic stability than the esters. However, the esters have acceptable hydrolytic stability and in particular have a hydrolytic stability greater than that of aspartame. Also, in terms of sweetness intensity, the esters tend to have a greater sweetness intensity.

The phenyl group R of the esters or amides of the present invention has the formula:

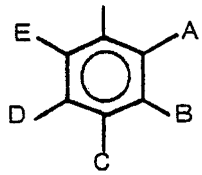

wherein A, B, C, D and E are H, OH, F, Cl, Br or $C_1$—$C_4$ alkyl, hydroxyalkyl or alkoxy. Preferred groups R are those where A, B, C, D and E are all H or where one of A, B, C, D and E is OH or F. Particularly preferred groups R are phenyl (A, B, C; D and E are H), p-hydroxyphenyl (C is OH; A, B, D and E are H) and o-fluorophenyl (A is F; B, C, D and E are H).

The terminal group R' can be selected from a variety of bicyclic radicals. The first group of such radicals have the formula (a):

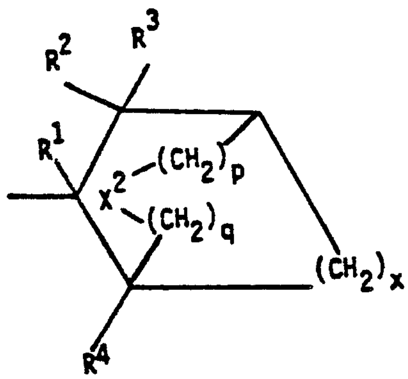

(a)

wherein $R^1$, $R^2$, $R^3$, and $R^4$ are H or $C_1$—$C_4$ alkyl, hydroxyalkyl or alkoxy; provided that at lest one of $R^2$, $R^3$ and $R^4$ are $C_1$—$C_4$ alkyl, hydroxyalkyl or alkoxy; $X^2$ is O; p and q are each 0, 1, 2 or 3; the sum of p + q being not greater than 3; and x is 1, 2 or 3. Preferably $R^2$, $R^3$ and $R^4$ are methyl or H; $R^1$ is preferably H; the sum of p + q is preferably 0; x is preferably 2. Especially preferred radicals of formula (a) are *alpha*-7-oxa-fenchyl; and *beta*-7-oxa-fenchyl.

A second set of such radicals have the formula (b):

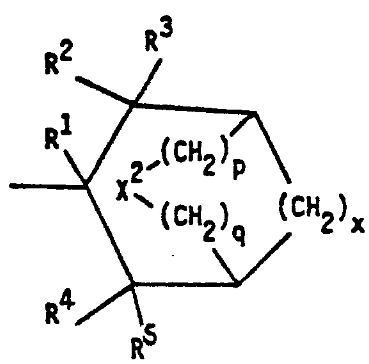

(b)

wherein $R^1$, $R^2$, $R^3$, $R^4$, $X^2$, p, q and x are defined as before; and $R^5$ is H or $C_1$—$C_4$ alkyl, hydroxyalkyl, or alkoxy. Preferably $R^2$, $R^3$, $R^4$ and $R^5$ are methyl or H; $R^1$ is preferably H; the sum of p + q is preferably 0; x is preferably 2.

A third set of such radicals have the formula (c):

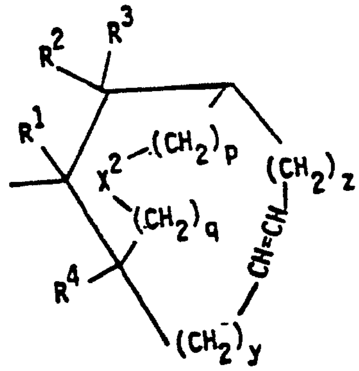

(c)

wherein $R^1$, $R^2$, $R^3$, $R^4$, $X^2$, p and q are defined as before; and y and z are 0, 1 or 2 and the sum of y + z is no greater than 2. Preferably, $R^2$, $R^3$ and $R^4$ are H or methyl; $R^1$ is preferably H; the sum of p + q is preferably 0; the sum of y + z is preferably 0 or 1.

B. *Sweetness Intensity of Alpha-L-Aspartyl-D-Phenylglycine Esters and Amides*

The sweetness intensity of the esters and amides of the present invention relative to sucrose can be determined according to the following procedure:

Male subjects are chosen at random from a group of about 20 persons who have previously been selected on the basis of proven tasting acuity, i.e., persons who could easily recognize the four basic tastes (sweet, sour, bitter and salty) and who are adept at quantifying their own physiological response numerically. The subjects are asked to taste and expectorate about 10 ml of a test sample (temperature of about 22°C) having dissolved therein the ester of amide. The subjects are then asked to compare the sweetness of the test sample with five standard samples which contain increasing amounts of sucrose. The standard samples are letter coded A, B, C, D and E and are designated on a ballot by a closed linear scale. Sweetness intensity of the test sample is recorded by the subject making a mark on the linear scale at a point he considers equal in sweetness among the standard samples; interpolation between standards is encouraged. After completion of the panel, a five point numeric scale is superimposed on the linear scales to obtain numerical data; data is averaged and recorded to the nearest 0.25 unit.. Equivalent sucrose sweetness is determined by referring to graphs of (w/v) sucrose concentration in the standard samples versus a linear numeric scale.

Sweetness intensity is calculated by dividing the concentration (w/v) of perceived sweetness by the concentration (w/v) of the ester or amide required to produce that sweetness. The five point scale with standard samples ranging from 1.37% (0.040 *M*) to 11.97% (0.35 *M*) sucrose is used for sweetness intensity testing. The test sample is prepared at a concentration which would be equal to about 8—10% sucrose.

The sweetness intensity of the esters and amides of the present invention is presented in the following table:

| R Group | Type | R′ Group | Sweetness (× Sucrose) |
|---|---|---|---|
| Phenyl | Ester | *alpha*-7-oxa-fenchyl | 1000* |

* based on informal panel testing

C. *Synthesis of alpha-L-aspartyl-D-phenylglycine esters and amides*

The *alpha*-L-aspartyl-D-phenylglycine esters of the present invention can be synthesized acording to the following 4-step reaction scheme:

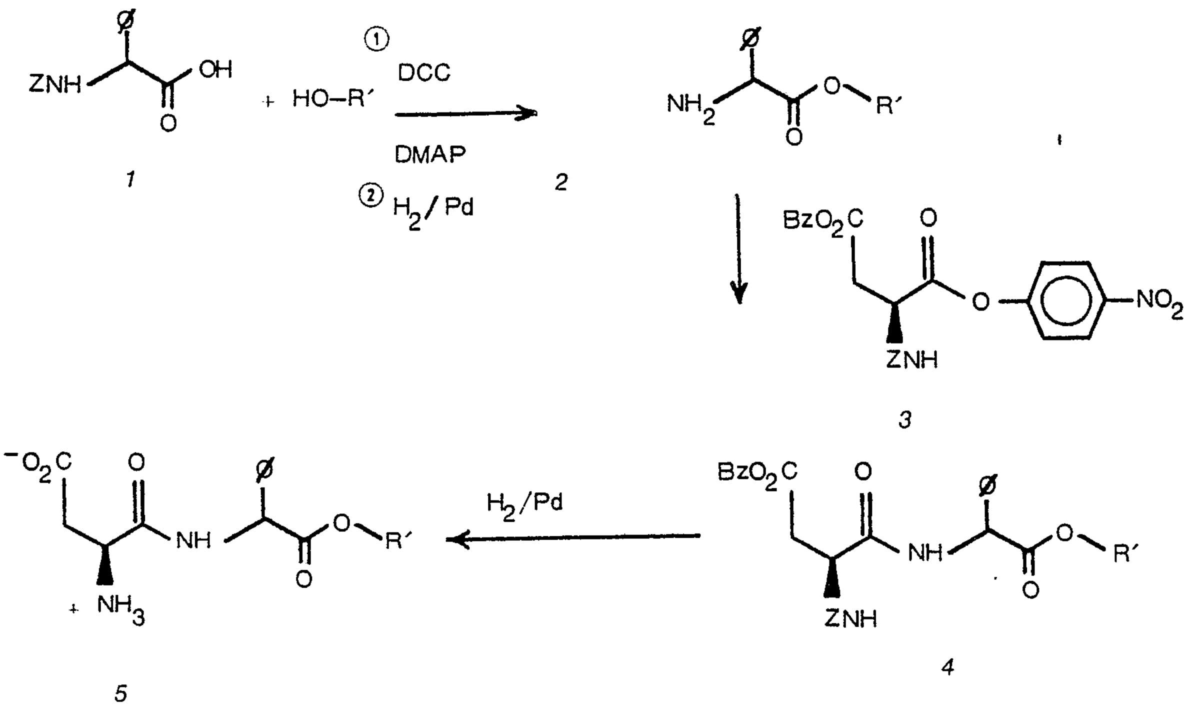


1 2 3 4 5

In the first step, carbobenzyloxy (Z) protected D-phenylglycine *1* is coupled with alcohol R'OH using dicyclohexylcarbodiimide (DCC)/dimethylaminopyridine (DMAP). In the second step, the ester formed in sep 1 is a hydrogenated over palladium to remove the protecting group to form the phenylglycine ester *2*. In the third step, ester *2* is coupled to the protected activated L-aspartic ester *3* to form the protected L-aspartyl-D-phenylglycine ester *4*. In the fourth step, the protecting groups are removed by hydrogenation of ester *4* over palladium to yield sweetener *5*.

Alcohols R'OH used in this synthesis are made according to the process disclosed in EP—A—0,168,882. This process involves the following 4-step reaction scheme:

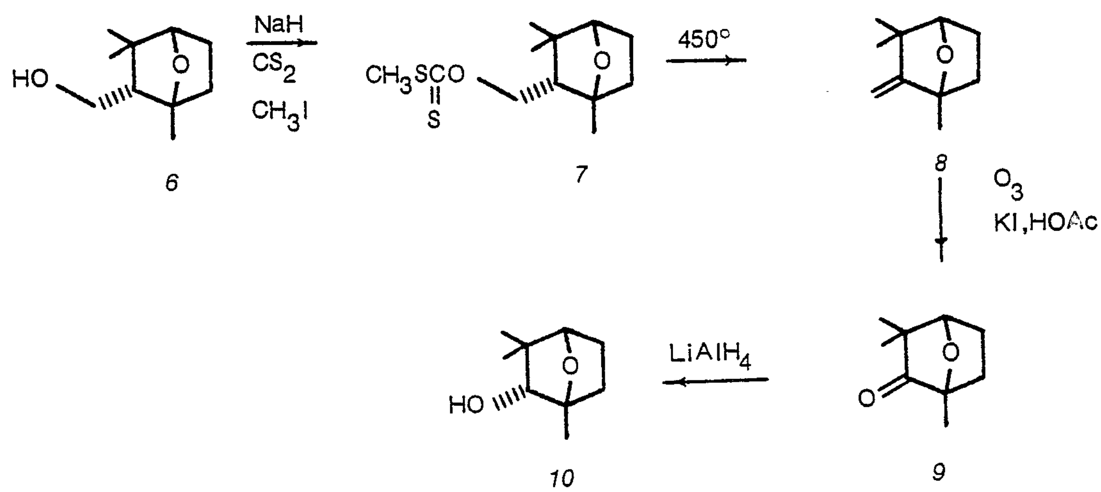


6 7 8 9 10

In the first step, alcohol *6* is converted to the xanthate ester *7* by using NaH, carbon disulfide and methyl iodide. In the second step, xanthate ester *7* is thermally decomposed to the methylene substituted bicyclic compound *8*. In the third step, bicyclic compound *8* is converted to ketone *9* by using ozone, KI and acetic acid. In the fourth step, ketone *9* is reduced to alcohol *10*.

Syntheses of specific *alpha*-L-aspartyl-D-phenylglycine esters are as follows:

Example 1
alpha-7-oxa-Fenchyl ester

*Step 1:*
*N-Carbobenzyloxy-D-phenylglycine-(±)-alpha-7-oxa-fenchyl ester*
a. *N-Carbobenzyloxy-D-phenylglycine*

To D-phenylglycine (50 g., 0.33 moles, Aldrich) is added 82 ml of 4 *N* NaOH. The mixture is cooled to 0°C and carbobenzoxy chloride (51 ml, 0.36 moles) is added dropwise. Additional NaOH is added as needed to keep the reaction mixture basic. After stirring for 10 minutes, 200 ml of $H_2O$ is added. After 10 more minutes, the solution is filtered. The clear filtrate is extracted twice with ether and is then adjusted to pH 3 with 5 *N* HCl. The resulting precipitate is filtered, washed twice with $H_2O$ and then dried. The crude product is dissolved in ethyl acetate and then filtered. The filtrate is evaporated and the resulting solid crystallized from ethyl acetate/hexane.

b. *(±)-alpha-7-oxa-Fenchol*

(±)-*alpha*-7-oxa-Fenchol is prepared according to the procedure of Example 2, Step 1b.

c. *N-Carbobenzyloxy-D-phenylglycine-(±)-alpha-7-oxa-fenchyl ester*

The N-carbobenzyloxy-D-phenylglycine (20 g, 0.07 moles) from step 1a is dissolved in about 150 ml of dry methylene chloride. The (±)-*alpha*-7-oxa-fenchol (10.9 g, 0.07 moles) from step 1b and N,N'-dicyclohexylcarbodiimide (17.3 g, 0.083 moles) are then added after cooling the solution to 0°C. The mixture thickens; additional methylene chloride (about 150 ml) is added. When the mixture becomes more uniform, it is then chilled to −65°C. 4-Dimethylaminopyridine is then added and the mixture stirred at −60° to −65°C for 1 hour. The cooling bath is then changed to carbon tetrachloride/dry ice to maintain the mixture at −23°C for 3 hours. The precipitated N,N'-dicyclohexylurea is filtered off. The filtrate is successively washed with chilled $H_2O$, 0.1 *N* HCl, 2% $NaHCO_3$, $H_2O$ and brine. The filtrate is dried over $MgSO_4$, filtered and then evaporated.

*Step 2:*
*D-Phenylglycine-(±)-alpha-7-oxa-fenchyl ester*

To a Parr flask is added 5% palladium on charcoal (200 mg). The crude ester (28.8 g) from step 1c in about 200 ml of methanol is then added. The contents of the flask are hydrogenated for 5 hours. Additional 5% palladium on charcoal (200 mg) plus 10% palladium on charcoal (100 mg) is added to the flask and hydrogenation is continued overnight. The contents of the flask are then filtered and evaporated to yield the crude product. This crude product is dissolved in 0.1 *N* HCl and is extracted twice with ether to remove non-basic impurities. The aqueous layer is adjusted to pH 9—10 with NaOH and is then extracted 3 times with ether. The combined extracts are successively washed with $H_2O$ and brine, and then dried over $MgSO_4$. The dried extracts are filtered and then evaporated to give the desired ester.

*Step 3:*
*beta-Benzyl-N-carbobenzyloxy-L-aspartyl-D-phenylglycine-(±)-alpha-7-oxa-fenchyl ester*
a. *beta-Benzyl-N-carbobenzyloxy-L-aspartyl-p-nitrophenyl ester*

To a 1000 ml 3-neck flask is added *beta*-benzyl-N-carbobenzyloxy-L-aspartic acid (50 g, 0.14 moles, Bachem Inc.), p-nitrophenol (23.5 g, 0.17 moles) and about 350 ml of ethyl acetate. This mixture is stirred and then 4-dimethylaminopyridine (1.0 g) and N,N'-dicyclohexylcarbodiimide (28.5 g, 0.14 moles) is added. The solution becomes warm; after 4 hours, the reaction is complete as measured by thin layer chromatography. The solution is then filtered to remove precipitated N,N'-dicyclohexylurea and then extracted 9 times with saturated $NaCO_3$ solution, then 2 times with saturated NaCl solution. The extracted solution is dried over $Na_2SO_4$ and then concentrated to yield the crude ester. This concentrated solution is dissolved in hot ethanol and then seeded. The concentrated solution is allowed to fully crystallize at room temperature and is then cooled with ice. The crystals are filtered and then washed with cold ethanol.

b. *beta-Benzyl-N-carbobenzyloxy-L-aspartyl-D-phenylglycine-(±)-alpha-7-oxa-fenchyl ester*

The p-nitrophenyl ester from step 3a (19.6 g, 0.041 moles) is dissolved in 100 ml of dry tetrahydrofuran (THF) and is chilled to 0°C. The 7-oxa-fenchyl ester from step 2 (11.8 g, 0.041 moles) is added and the reaction mixture is then stirred at 0°C for 1 hour. The reaction mixture is stirred overnight at room temperature and then the THF is evaporated. The residue is partitioned between ethyl acetate and $H_2O$. The organic layer is successively washed with cold 10% $Na_2CO_3$, $H_2O$, and brine, and then dried over $MgSO_4$. The dried solution is filtered and then evaporated to give the crude product. This crude product is purified by silica gel chromatography first with 2% acetone/chloroform solvent and then with 25% ethyl acetate/hexane solvent.

*Step 4:*
*alpha-L-Aspartyl-D-phenylglycine-(−)-alpha-7-oxa-fenchyl ester*

The purified ester from step 3b (7 g, 0.011 moles) is dissolved in 150 ml of methanol and is then hydrogenated over 5% palladium on charcoal (300 mg) for 22 hours. A second portion of the purified ester

from step 3b (8 g, 0.013 moles) is hydrogenated over 10% palladium on charcoal (300 mg) for 5 hours. The catalyst is filtered off and the solvent evaporated for a combined yield of the desired sweetener.

In certain instances, use of carbobenzyloxy protected D-phenylglycine can cause partial racemization at the asymmetric carbon of the phenylglycine moiety during formation of ester *2*. Racemization can be minimized by using o-nitrophenylsulfenyl (o-Nps) protected D-phenylglycine to form ester *2* according to the following reactions:

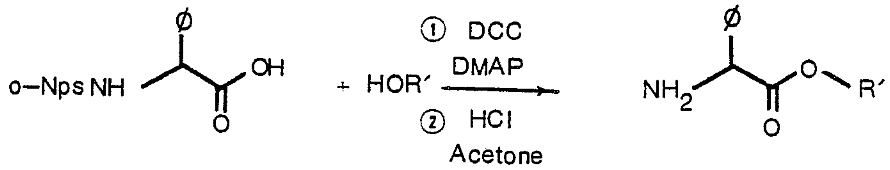


2

Ester *2* can be converted to the desired ester *5* by the previously described procedure.

Synthesis of specific esters *5* using o-nitrophenylsulfenyl protected D-phenylglycine are as follows:

Example 2
alpha-7-oxa-Fenchyl ester

*Step 1:*
*o-Nitrophenylsulfenyl-D-phenylglycine-(±)-alpha-7-oxa-fenchyl ester*
*a: o-Nitrophenylsulfenyl-D-phenylglycine*

D-phenylglycine (51 g, 0.34 moles, Aldrich) was dissolved in 180 ml of 2*N* NaOH and 200 ml of dioxane. Then o-nitrophenylsulfenyl chloride (64 g, 0.34 moles) was added in small portions over 1 hour with simultaneous addition of 180 ml of 2*N* NaOH. The reaction mixture was stirred for 2 hours and then diluted with 500 ml of $H_2O$. The mixture was filtered and the solids washed with $H_2O$. The filtrate was acidified with $H_2SO_4$ and then extracted 3 times with ether. The combined extracts were successively washed with $H_2O$ and brine, dried over $Na_2SO_4$ and then evaporated. The crude product was then recrystallized from ethyl acetate/hexane. Yield: 64.5 g. The purified product was characterized by NMR. $[\alpha]_D = -179.5°$ (C 0.4, methanol).

*b: (±)-alpha-7-oxa-Fenchol*
*(1): (±)-endo-1,3,3-Trimethyl-7-oxabicyclo[2.2.1]heptane-2-methanol*

Geraniol was converted to (±)-endo-1,3,3-trimethyl-7-oxabicyclo[2.2.1]heptane-2-methanol using thallium (III) perchlorate according to the procedure described in Yamada et al., *J. Chem. Soc. Chem. Comm.,* (1976), page 997.

*(2): S-methyl xanthate ester of (±)-endo-1,3,3-trimethyl-7-oxabicyclo[2.2.1]heptane-2-methanol*

(±)-endo-1,3,3-Trimethyl-7-oxabicyclo[2.2.1]heptane-2-methanol from step (1) (2.1 g, 0.013 moles) was slowly added to a suspension of NaH (0.90 g., 0.038 moles) in 100 ml of THF at 0°C under argon. After stirring at 0°C for 5 minutes, the reaction mixture was refluxed for 2 hours. Carbon disulfide (2.9 g, 0.038 moles) was added dropwise and the reaction mixture was refluxed for 1 hour. Methyl iodide (5.35 g, 0.037 moles) was then added dropwise and the reaction mixture was refluxed for an additional 2 hours. At this point, the reaction mixture was cooled to room temperature, $H_2O$ was slowly added until two phases formed, the layers were separated, and the aqueous layer was extracted with ether. The organic layers were combined, washed successively with $H_2O$ and brine, and then dried over $MgSO_4$. Evaporation of the solvent and vacuum distillation of the residue afforded the xanthate as an amber oil. Yield: 2.78 g. The distilled product was characterized by NMR.

*(3): (±)-1,3,3-Trimethyl-2-methylidine-7-oxabicyclo[2.2.1)heptane*

The xanthate ester from step (2) (2.78 g, 0.011 moles) was pyrolyzed in the vapor phase at 450°C, 0.1 mm pressure using a glass tube packed with glass beads heated by a cylindrical furnace. The product was collected using two traps connected in series, both cooled to −78°C. Yield: 1.27 g. The crude product was characterized by NMR.

*(4): (±)-1,3,3-Trimethyl-7-oxabicyclo[2.2.1]heptane-2-one*

A stream of 3—5% ozone in oxygen was passed through a solution of (±)-1,3,3-trimethyl-2-methylidine-7-oxabicyclo[2.2.1]heptane from step (3) (1.20 g, 0.007 moles) in 35 ml. of methanol at −78°C until the solution became light blue (ozone saturation). The excess ozone was removed by purging the cold reaction mixture with oxygen for 15 minutes. The cold reaction mixture was then poured onto a stirred solution of 15 ml of methanol, 4 ml of glacial acetic acid, and 8 g of sodium iodide and stirred for 30 minutes. Sodium thiosulfate solution (0.1 *N*) was added to decompose the liberated iodine. Saturated sodium bicarbonate solution was then added until the mixture was slightly basic (pH 7.5). The aqueous

mixture was extracted with ether, the extract washed with brine, and then dried over $Na_2SO_4$. Evaporation of the solvent afforded the product which was characterized by NMR. Yield: 1.12 g.

*(5): (±)-endo-2-Hydroxy-1,3,3-trimethyl-7-oxabicyclo[2.2.1]heptane ((±)-alpha-7-oxa-fenchol)*

A 1 *M* solution of lithium aluminum hydride in ether (15 ml, 0.015 moles) was added dropwise to a solution of (±)-1,3,3-trimethyl-7-oxabicyclo[2.2.1]heptane-2-one from step (4) (1.10 g, 0.006 moles) in 50 ml of THF at 0°C. The reaction mixture was stirred for 30 minutes, and then quenched by the careful addition of saturated $Na_2SO_4$ solution. The resulting white precipitate was removed by vacuum filtration and washed with ether. The filtrate was evaporated, affording the product as a colorless oil which was characterized by NMR. Yield: 0.82 g.

*c: o-Nitrophenylsulfenyl-D-phenylglycine-(±)-alpha-7-oxafenchyl ester*

The purified o-Nps-D-phenylglycine from step 1a (1.44 g, 0.005 moles) and (±)-*alpha*-7-oxafenchol from step 1b (0.74 g, 0.005 moles) were dissolved in 50 ml of $CH_2Cl_2$ and cooled to −65°C. N,N'-dicyclohexylcarbodiimide (1.00 g, 0.005 moles) was added and the mixture then stirred for 20 minutes. A catalytic amount of 4-dimethylaminopyridine (33 mg) was added and then this reaction was stirred at −65°C for 1 hour. The rection mixture was then gradually warmed to −23°C ($CCl_4$/ice bath) and stirred for 3 hours. The mixture was then filtered and the filtrate washed successively with $H_2O$, 2% $Na_2CO_3$, $H_2O$, and brine. The washed filtrate was dried over $MgSO_4$, filtered and then concentrated to give the crude product. The crude product was purified by flash chromatography on silica gel using 25% ethyl acetate/hexane as the eluting solvent. The purified product was characterized by NMR. Yield: 1.18 g.

*Step 2:*
*D-Phenylglycine-(±)-alpha-7-oxa-fenchyl ester*

The purified o-Nps-D-phenylglycine-(±)-*alpha*-7-oxa-fencyl ester from step 1b (1.10 g, 0.0025 moles) was dissolved in 50 ml of acetone and 5*N* HCl (0.5 ml) was added. The reaction mixture was stirred for 15 minutes and then the acetone was evaporated. The residue was dissolved in 0.1*N* HCl, was extracted with ether to remove non-basic impurities and was then adjusted to pH 10 with NaOH. The alkaline solution was extracted with ethyl acetate 3 times. The combined extracts were successively washed with $H_2O$ and brine, dried over $MgSO_4$, and then evaporated to give the desired ester which was characterized by NMR. Yield: 0.55 g.

*Step 3:*
*beta-Benzyl-N-carbobenzyloxy-L-aspartyl-D-phenylglycine-(±)-alpha-7-oxa-fenchyl ester.*

By a procedure similar to that of Example 1, Step 3, the ester from step 2 was converted to the diprotected L-aspartyl-D-phenylglycine-(±)-*alpha*-7-oxa-fenchyl ester. Yield: 0.91 g.

*Step 4:*
*alpha-L-Aspartyl-D-phenylglycine-alpha-7-oxa-fenchyl ester*

By a procedure similar to that of Example 1, Step 4, the diprotected ester from step 3 was converted to a mixture of diastereomers from which the desired sweetener (either (+) or (−) oxa-fenchyl ester) was isolated by semi-preparative high performance liquid chromatogrpahy using a Whatman Magnum 9 ODS—3 column and 0.01 *M* ammonium acetate in methanol/water (50/50), pH adjusted to 5.4 with acetic acid, as the eluting solvent. The sweetener identity was confirmed by NMR. Sweetness intensity: approximately 1000X based on informal panel testing.

The *alpha*-L-aspartyl-D-phenylglycine amides of the present invention can also be synthesized according to the previously described schemes for the esters by using a primary amine R'$NH_2$ instead of the alcohol. Amines R'$NH_2$ used in this synthesis can be obtained from the respective ketone *4* by the oxime procedure described in U.S. Patent 4,411,925 to Brennan et al., issued October 25, 1983, especially column 12, line 55 to column 20, line 9, and Example 47. See also EP—A—0,168,112 Example 10, for the synthesis of an amide according to this reaction scheme.

The amides of the present invention can also be synthesized according to the following alternative 4-step reaction scheme:

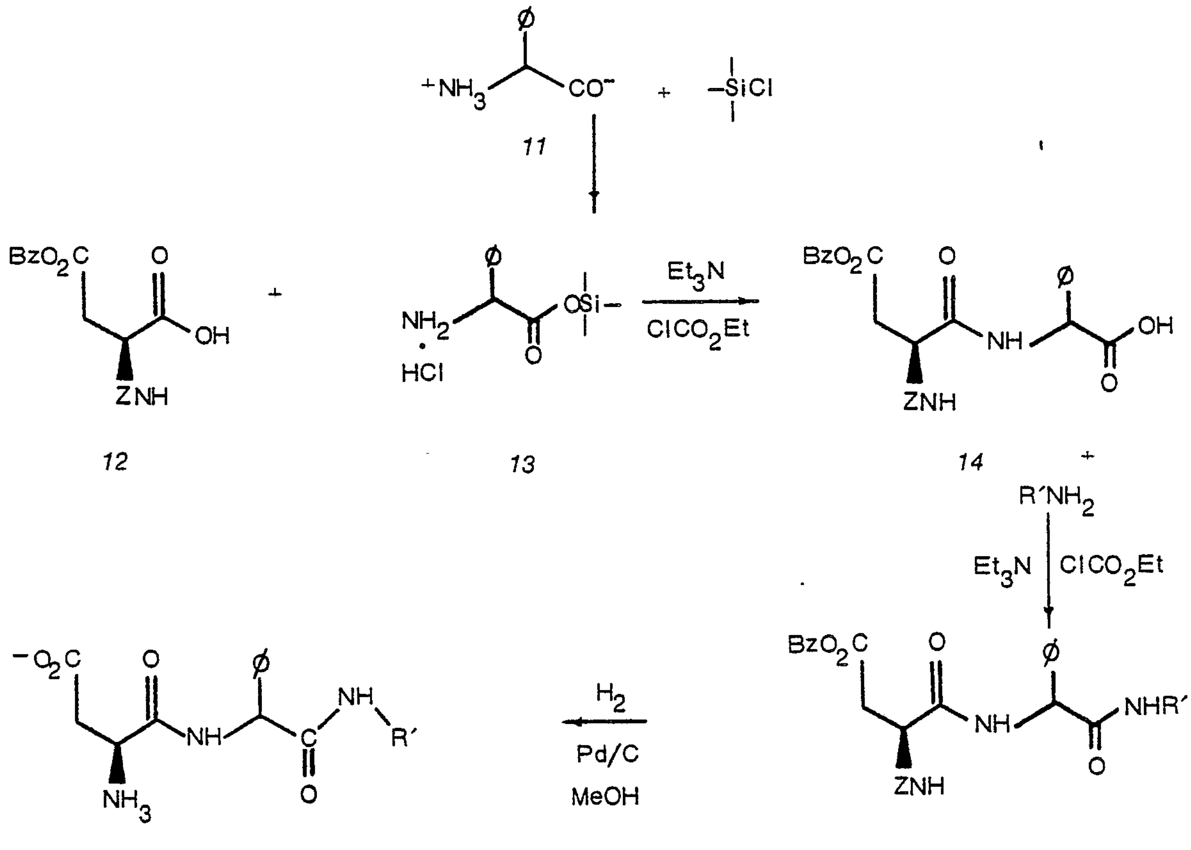


11 12 13 14 15 16

In the first step, D-phenylglycine *11* is reacted with trimethylsilylchloride to form the silyl ester *12*. In the second step, sillyl ester *12* is coupled to diprotected L-aspartic acid ester *13* using triethylamine and ethyl chloroformate to form diprotected amide *14*. In the third step, amine $R'NH_2$ is coupled to diprotected amide *14* using triethylamine and ethyl chloroformate to form diprotected amide *15*. In the fourth step, the protecting groups are removed by hydrogenation of amide *15* over palladium to yield sweetener *16*. See EP—A—0,168,112 Example 12, for the synthesis of an amide according to this alternative reaction scheme.

The *alpha*-L-aspartyl-D-p-hydroxyphenylglycine esters of the present invention can be synthesized according to Example 13 of EP—A—0,168,112.

D. *Uses of alpha-L-aspartyl-D-phenylglycine esters and amides*

The esters of amides of the present invention can be used to sweeten a variety of edible materials. Also, mixtures of these esters or amides with other sweeteners, in particular, mixtures of these esters or amides with saccharin or its non-toxic salts can be used. As used herein, "non-toxic salts of saccharin" means those salts of saccharin with physiologically acceptable cations such as sodium, potassium, calcium or ammonium. The mixtures of the present esters or amides with saccharin can be in a ratio (sweetness equivalent basis) of from 2:1 to 1:9, and preferably from 1:1 to 1:4. Mixtures of the present esters and amides with sweeteners other than saccharin can also be used. Examples of such sweeteners include Acesulfam; the *alpha*-L-aspartyl-L-phenylalanine lower alkyl esters disclosed in US—A—3,492,131 to Schlatter, issued January 27, 1970, in particular the methyl ester known as aspartame; the *alpha*-L-aspartyl-L-1-hydroxymethylalkyl amides disclosed in US—A—4,338,346 to Brand, issued July 6, 1982; the *alpha*-L-aspartyl-L-1-hydroxyethylalkyl amides disclosed in US—A—4,423,029 to Rizzi, issued December 27, 1983; the *alpha*-L-aspartyl-D-alanine amides disclosed in US—A—4,411,925 to Brennan et al., issued October 25, 1983 and the *alpha*-L-aspartyl-D-serine amides disclosed in US—A—4,399,263 to Brennan et al., issued August 16, 1983. Low calorie mixtures can also be formulated which contain esters or amides of the present invention with sucrose.

The esters and amides of the present invention, including mixtures thereof with other sweeteners, are useful for sweetening a variety of food products, such as fruits, vegetables, juices, cereals, meat products such as ham or bacon, sweetened milk products, egg products, salad dressings, ice creams and sherbets, gelatins, icings, syrups, cake mixes and frostings. In particular, these sweeteners are useful for sweetening a variety of beverages such as lemonade, coffee, tea, and particularly carbonated beverages. The sweeteners of the present invention can also be used to sweeten dentifrices, mouthwashes, and chewing gums, as well as drugs such as liquid cough and cold remedies. As an alternative to direct addition of the

esters and amides of the present invention to the foregoing edible materials, sweetener concentrates can be prepared using these esters and amides in, for example, granular or liquid form. These concentrates can then be conventionally metered into foods, beverages and the like as desired by the user.

The esters and amides of the present invention are stable substances that can be used in a variety of physical forms such as powders, granules, tablets, syrups, pastes, solutions and the like. Liquid or solid ingestible carriers such as water, glycerol, starch, sorbitol, salts, citric acids, celulose and other suitable non-toxic subsances can also be used. These sweetening agents can be readily used in pharmaceutical compositions to impart a sweet taste.

The ester and amide sweeteners of the present invention are used in amounts sufficient to provide a sweet taste of the desired intensity for orally ingested products. The amount of the sweetener added will generally depend upon commercial needs as well as individual sweetness sensitivities.

Specific Embodiments of Oral Products Containing Alpha-L-Aspartyl-D-Phenylglycine Esters

A. Beverage

Mixtures of the *alpha*-7-oxa-fenchyl ester sweetener of Example 2 with other sweeteners are used in cola beverages that are formulated as follows:

| Ingredients | Embodiment 1 (%) | Embodiment 2 (%) |
|---|---|---|
| $H_3PO_4$ | 0.06 | 0.06 |
| Caramel color | 0.25 | 0.25 |
| Flavor | 0.0032 | 0.0032 |
| Saccharin | 0.020 | 0.011 |
| Aspartame | 0.005 | 0.015 |
| Fenchyl ester | 0.0005 | 0.0036 |
| $CO_2$ | 3.5 (volumes) | 3.5 (volumes) |

B. *Toothpaste*

The following toothpaste formulation is within the scope of the present invention:

| Ingredient | Wt. % |
|---|---|
| Calcium pyrophosphate | 40.00 |
| Sorbitol (70% aqueous solution) | 20.40 |
| Glycerine | 10.20 |
| Sodium coconut monoglyceride sulfonate | 0.80 |
| Sodium carboxymethyl cellulose | 1.20 |
| Sodium coconut alkyl sulfate (20% active) | 2.30 |
| Sodium fluoride | 0.22 |
| Sweetener (Example 2) | 0.016 |
| Flavor | 0.90 |
| Red urea formaldehyde agglomerates | 0.65 |
| Water and minor ingredients | 0.65 |
| Water and minor ingredients | Balance |

C. *Mouthwash*

A mouthwash according to the present invention is prepared by co-dissolving the following ingredients:

| Ingredient | Percent by Weight |
|---|---|
| Glycerine | 10.00 |
| Ethyl alcohol | 17.00 |
| Cetyl pyridinium chloride | 0.05 |
| Sorbitan monooleate polyoxyethylene | 0.13 |
| Flavor (Oil of Wintergreen) | 0.09 |
| Sweetening agent* | 0.02 |
| Water and minor ingredients | Balance |

* Sweetener of Example 2, Hydrochloride salt

D. Dentifrice

A gel dentifrice having the following formulation is prepared by conventional means:

| Ingredients | Percent by Weight |
|---|---|
| Silica xerogel | 12.00 |
| Silica aerogel | 5.00 |
| Hydroxyethyl cellulose | 1.50 |
| Glycerine | 34.76 |
| Stannous fluoride | 0.41 |
| Flavor (Wintergreen) | 0.95 |
| Color (FD & C Blue #1) | 0.03 |
| 21% sodium lauryl sulfate-79% glycerine mixture | 6.00 |
| Sweetener* | 0.012 |
| Water and minor ingredients | Balance |

* Example 2, Calcium salt.

The above composition is prepared by blending and deaerating the listed ingredients in standard fashion.

E. *Chewing Gum*

A chewing gum is prepared by replacing the sucrose normally added to a chewing gum with the sweeteners of the present invention. A gum base is prepared from:

| Ingredients | Weight in Grams |
|---|---|
| 60% latex | 18 |
| Hydrogenated rosin esters | 44 |
| Paracumarine resin | 7.5 |
| Candellila wax | 6 |
| Glyceryl tristearate | 2.5 |
| Ethyl cellulose | 2 |
| Calcium carbonate | 20 |

The gum base is used with the sweeteners of the present invention to prepare a chewing gum having a greatly reduced sugar content.

| Ingredients | Percent by Weight |
|---|---|
| Gum base | 68 |
| Sweetener* | 0.6 |
| Corn syrup | 16 |
| Flavor | 1 |

* Example 2

Chewing gum can also be prepared using other sweeteners of the present invention.

F. *Powdered Sweetener Concentrate*

| | |
|---|---|
| Sweetener of Example 1, Hydrochloride Salt | 6.4 mg. |
| Dextrose | 840 mg. |

One packet containing the foregoing ingredients will be the approximate equivalent of two teaspoons of sugar.

H. *Liquid Sweetener Concentrate*

| | Gm. % |
|---|---|
| Example 2, Hydrochloride salt | 0.12 |
| Benzoic acid | 0.1 |
| Methyl paraben | 0.05 |
| Water | Balance |

Ten drops provides the approximate sweetening power of one teaspoon of sugar.

## Claims

1. An ester or amide of *alpha*-L-aspartyl-D-phenylglycine characterized in that it has the formula:

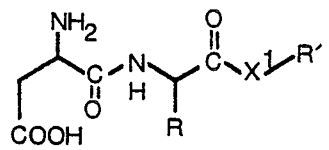

wherein the ester or amide is the L, D stereochemical isomer; wherein $X^1$ is O or NH; wherein R is a phenyl group having the formula:

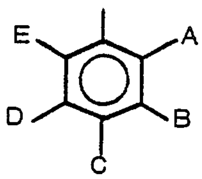

wherein A, B, C, D and E are H, OH, F, Cl, Br, or $C_1$—$C_4$ alkyl, hydroxyalkyl or alkoxy; and wherein R′ is selected from bicyclic radicals having formulas (a) (b) and (c):

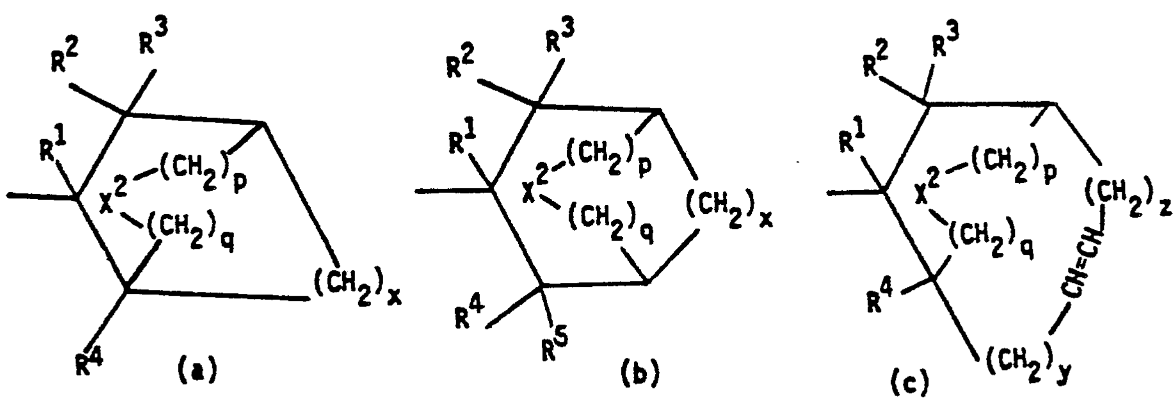

(a) (b) (c)

wherein $R^1$, $R^2$, $R^3$, $R^4$ and $R^5$ are H, or $C_1$—$C_4$ alkyl, hydroxyalkyl or alkoxy; provided that at least one of $R^2$, $R^3$, $R^4$ and $R^5$ is $C_1$—$C_4$ alkyl, hydroxyalkyl or alkoxy; $X^2$ is O; p and q are 0, 1, 2 or 3 and the sum of p + q is not greater than 3; x is 1, 2, or 3; y and z are 0, 1 or 2 and the sum of y + z is not greater than 2; and nontoxic salts thereof.

2. The ester or amide of Claim 1, charcterized in that C is OH and A, B, D and E are each H.

3. The ester or amide of Claim 1, characterized in that A, B, C, D and E are each H.

4. The ester or amide of any of Claims 1 to 3 characterized in that R′ is *alpha*-7-oxa-fenchyl or *beta*-7-oxa-fenchyl.

5. The ester of any of Claims 1 to 4 characterized in that $X^1$ is O.

6. A diasteromeric mixture of the ester or amide of any of Claims 1 to 5 characterized in that the L, D sterochemical isomer comprises at least 50% of the mixture.

7. The diastereomeric mixture of Claim 6 characterized in that the L, D stereochemical isomer comprises at least 70% of the mixture.

8. The diastereomeric mixture of Claim 7 characterized in that the L, D, stereochemical isomer comprises at least 95% of the mixture.

9. An orally ingestible composition characterized in that it comprises the ester or amide of any of Claims 1 to 8, and an ingestible carrier.

10. The composition of Claim 9 characterized in that it is a food or beverage.

11. The composition of Claim 10 characterized in that it is a carbonated beverage.

12. The ester or amide of any of Claims 1 to 8 characterized in that it is mixed with a sweetener selected from saccharin and *alpha*-L-aspartyl-D-phenylalanine lower alkyl esters in a ratio of from 2:1 to 1:9 on a sweetness equivalent basis.

**Patentansprüche**

1. Ein Ester oder Amid von alpha-L-Asparaginyl-D-phenylglycin, dadurch gekennzeichnet, daß er bzw. es die Formel:

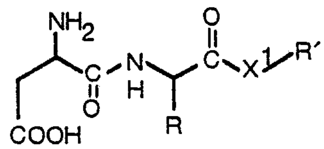

hat, worin der Ester oder das amid das stereochemische L,D-Isomer ist; worin $X^1$ O oder NH ist; worin R eine die Formel:

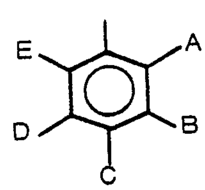

aufweisende Phenylgruppe ist, wobei A, B, C, D und E für H, OH, F, Cl, Br oder $C_1$—$C_4$-Alkyl, -Hydroxyalkyl oder -Alkoxy stehen; und worin R′ aus bicyclischen Resten der Formeln (a), (b) und (c):

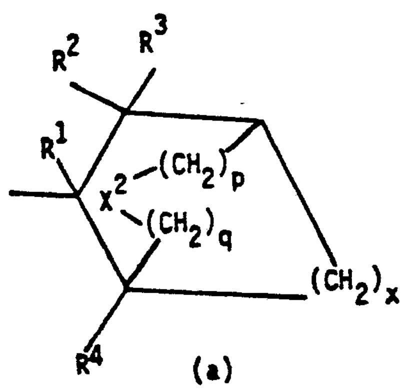

(a)

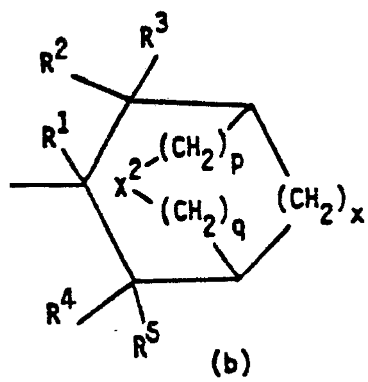

(b)

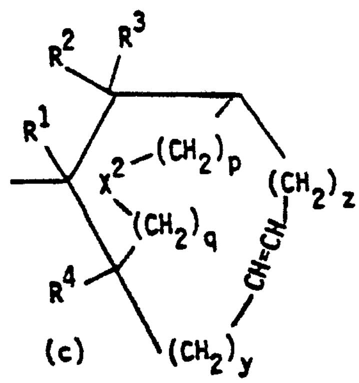

(c)

ausgewählt ist, wobei $R^1$, $R^2$, $R^3$, $R^4$ und $R^5$ für H oder $C_1$—$C_4$-Alkyl, -Hydroxyalkyl oder -Alkoxy stehen; mit der Maßgabe, daß wenigstens einer der Reste $R^2$, $R^3$, $R^4$ und $R^5$ $C_1$—$C_4$-Alkyl, -Hydroxyalkyl oder -Alkoxy ist; $X^2$ 0 ist; p und q 0, 1, 2 oder 3 sind, und die Summe von p + q nicht größer als 3 ist; x 1, 2 oder 3 ist; y und z 0, 1 oder 2 sind, und die Summe von y + z nicht größer als 2 ist; und die nicht-toxischen Salze davon.

2. Der Ester oder das Amid nach Anspruch 1, dadurch gekennzeichnet, daß C OH ist, und A, B, D und E jeweils H sind.

3. Der Ester oder das Amid nach Anspruch 1, dadurch gekennzeichnet, daß A, B, C, D und E jeweils H sind.

4. Der Ester oder das Amid nach einem der Ansprüche 1 bis 3, dadurch gekennzeichnet, daß R′ alpha-7-Oxa-fenchyl oder beta-7-Oxa-fenchyl ist.

5. Der Ester nach einem der Ansprüche 1 bis 4, dadurch gekennzeichnet, daß $X^1$ für O steht.

6. Ein diastereomeres Gemisch des Esters oder Amids nach einem der Ansprüche 1 bis 5, dadurch gekennzeichnet, daß das stereochemische L,D-Isomer wenigstens 50% des Gemisches ausmacht.

7. Das diastereomere Gemisch nach Anspruch 6, dadurch gekennzeichnet, daß das stereochemische L,D-Isomer wenigstens 70% des Gemisches ausmacht.

8. Das diastereomere Gemisch nach Anspruch 7, dadurch gekennzeichnet, daß das stereochemische L,D-Isomer wenigstens 95% des Gemisches ausmacht.

9. Eine oral einnehmbare Zusammensetzung, dadurch gekennzeichnet, daß sie den Ester oder das Amids nach einem der Ansprüche 1 bis 8 und einen einnehmbaren Träger enthält.

10. Die Zusammensetzung nach Anspruch 9, dadurch gekennzeichnet, daß sie ein Lebensmittel oder Getränk ist.

11. Die Zusammensetzung nach Anspruch 10, dadurch gekennzeichnet, daß sie ein kohlensäurehältiges Getränk ist.

12. Der Ester der das Amid nach einem der Ansprüche 1 bis 8, dadurch gekennzeichnet, daß er bzw. es mit einem Süßstoff, ausgewählt aus Saccharin und alpha-L-Asparaginyl-D-phenylalanin-niedrigalkylestern, in einem Verhältnis von 2:1 bis 1:9 auf einer Süßeäquivalentbasis vermischt ist.

## Revendications

1. Ester ou amide d'α-L-aspartyl-D-phénylglycine, caractérisé en ce qu'il a pour formule:

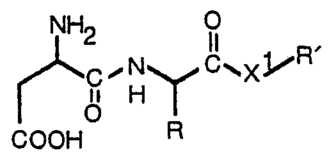

dans laquelle l'ester ou l'amide est l'isomère stéréochimique L,D; dans laquelle $X^1$ est O ou NH; dans laquelle R est un groupe phényle ayant pour formule:

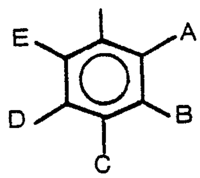

dans laquelle A, B, C, D et E sont H, OH, F, Cl, Br ou un alkyle, un hydroxyalkyle ou un alcoxy en $C_1$—$C_4$; et dans laquelle R' est choisi parmi les radicaux bicycliques ayant pour formules (a), (b) et (c):

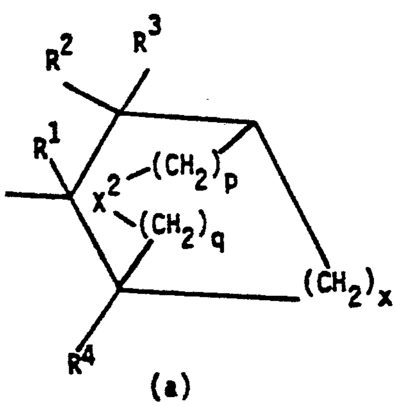
(a)

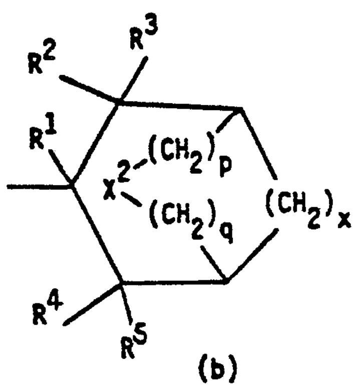
(b)

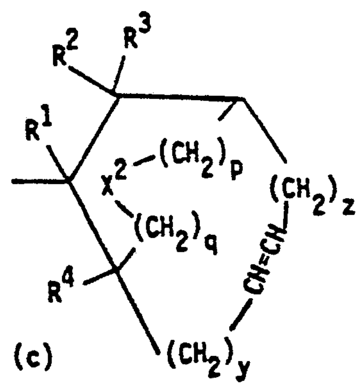
(c)

dans lesquelles $R^1$, $R^2$, $R^3$, $R^4$ et $R^4$ sont H, ou un alkyle, un hydroxyalkyle ou un alcoxy en $C_1$—$C_4$; à la condition qu'au moins un parmi $R^2$, $R^3$, $R^4$ et $R^5$ soit un alkyle, un hydroxyalkyle ou un alcoxy en $C_1$—$C_4$; $X^2$ est O; p et q sont égaux à 0, 1, 2 ou 3 et la somme de p + q n'est pas supérieure à 3; x est égal à 1, 2 ou 3; y et z sont égaux à 0, 1 ou 2, et la somme y + z n'est pas supérieur à 2; et des sels non toxiques de ceux-ci.

2. Ester ou amide selon la revendication 1, caractérisé en ce que C est OH et A, B, D et E sont chacun H.

3. Ester ou amide selon la revendication 1, caractérisé en ce que A, B, C, D et E sont chacun H.

4. Ester ou amide selon l'une quelconque des revendications 1 à 3, caractérisé en ce que R' est l'α-7-oxa-fenchyle ou le β-7-oxa-fenchyle.

5. Ester selon l'une quelconque des revendications 1 à 4, caractérisé en ce que $X^1$ est O.

6. Mélange diastéréoisomère de l'ester ou de l'amide selon l'une quelconque des revendications 1 à 5, caractérisé en ce que l'isomère stéréochimique L,D constitue au moins 50% du mélange.

7. Mélange diastéréoisomère selon la revendication 6, caractérisé en ce que l'isomère stéréochimique L,D constitue au moins 70% du mélange.

8. Mélange diastéréoisomère selon la revendication 7, caractérisé en ce que l'isomère stéréochimique L,D constitue au moins 95% du mélange.

9. Composition pour l'ingestion par voie orale, caractérisée en ce qu'elle comprend l'ester ou l'amide selon l'une quelconque des revendications 1 à 8 et un véhicule convenant pour l'ingestion.

10. Composition selon la revendication 9, caractérisée en ce qu'elle est un aliment ou une boisson.

11. Composition selon la revendication 10, caractérisée en ce qu'elle est une boisson gazeuse.

12. Ester ou amide selon l'une quelconque des revendications 1 à 8, caractérisé en ce qu'il est mélangé à un édulcorant choisi parmi la saccharine et les esters d'alkyle inférieur d'α-L-aspartyl-D-phénylalanine, dans un rapport de 2:1 à 1:9, sur une base d'équivalent de saveur sucrée.